Europäisches Patentamt

European Patent Office

Office européen des brevets

(19)

(11) Publication number: **0 190 816**
**B1**

# EUROPEAN PATENT SPECIFICATION

(12)

(45) Date of publication of the patent specification:
21.03.90

(51) Int. Cl.⁴: **C10G 3/00**

(21) Application number: 86300049.3

(22) Date of filing: 07.01.86

(54) **Process for converting oxygenates into liquid hydrocarbons.**

(30) Priority: 17.01.85 US 692261
14.05.85 US 733994

(43) Date of publication of application:
13.08.86 Bulletin 86/33

(45) Publication of the grant of the patent:
21.03.90 Bulletin 90/12

(84) Designated Contracting States:
BE DE FR GB IT NL

(56) References cited:
EP-A- 0 096 996
US-A- 4 471 147
US-A- 4 482 772
US-A- 4 506 106

(73) Proprietor: MOBIL OIL CORPORATION, 150 East 42nd
Street, New York New York 10017(US)

(72) Inventor: Gould, Ronald Michael, 223 Champion Way,
Sewell New Jersey 08080(US)
Inventor: Tabak, Samuel Allen, 204 East Pine Street,
Wenonah New Jersey 08090(US)

(74) Representative: Cooper, John Anthony et al, Mobil
Court 3 Clements Inn, London WC2A 2EB(GB)

EP 0 190 816 B1

## Description

This invention relates to a process for converting oxygenates, such as methanol and dimethyl ether (DME), into liquid hydrocarbons, and more particularly, to a process for converting the oxygenate feedstock catalytically into an intermediate lower olefinic stream and for oligomerizing the olefins to produce distillate and gasoline.

In order to provide an adequate supply of liquid hydrocarbons for use as synfuels or chemical feedstocks, various processes have been developed for converting coal and natural gas into gasoline, distillate and lubricants. A substantial body of technology has grown to provide oxygenated intermediates, especially methanol. Large scale plants can convert methanol and similar aliphatic oxygenates into liquid fuels, especially gasoline. However, the demand for heavier hydrocarbons has led to the development of processes for increasing the yields of diesel fuel by a multi-stage technique.

Recent developments in zeolite catalysts and hydrocarbon conversion processes have created interest in utilizing olefinic feedstocks for producing $C_5^+$ gasoline and diesel fuel, for example. In addition to the basic work derived from ZSM-5 type zeolite catalysts, a number of discoveries have contributed to the development of a new industrial process, known as Mobil Olefins to Gasoline/Distillate (MOGD) process. This process has significance as a safe, environmentally acceptable technique for utilizing feedstocks that contain lower olefins, especially $C_2$-$C_5$ alkenes, and may even supplant conventional alkylation units. In U.S. Patents 3,960,978 and 4,021,502, there is described conversion of $C_2$-$C_5$ olefins, alone or in admixture with paraffinic components, into higher hydrocarbons over crystalline zeolites having controlled acidity. Improved processing techniques for the MOGD system are described in U.S. Patents 4,150,062, 4,211,640 and 4,227,992.

Conversion of lower olefins, especially propene and butenes, over HZSM-5 zeolite is effective at moderately elevated temperatures and pressures. The conversion products are valuable as liquid fuels, especially the $C_5^+$ aliphatic and aromatic hydrocarbons. Olefinic gasoline is produced in good yield by the MOGD process and may be recovered or recycled to the reactor system for further conversion into distillate-range products. Operating details for typical MOGD units are described in U.S. Patents 4,445,031, 4,456,779 and 4,433,185.

In addition to their use as shape-selective components of oligomerization catalysts, the medium pore ZSM-5 type zeolites are useful also for converting methanol and other lower aliphatic alcohols or corresponding ethers into olefins. Particular interest has been directed to a catalytic process (MTO) for converting low cost methanol into valuable hydrocarbons rich in ethene and $C_3^+$ alkenes. Various processes of this type are described in U.S. Patents 3,894,107, 3,928,483, 4,025,571, 4,423,274, and 4,433,189. It is generally known that the MTO process can be optimized to produce a major fraction of $C_2$-$C_4$ olefins. Prior process proposals in this respect have included a separation section to recover ethene and other gases from byproduct water and $C_5^+$ hydrocarbon liquids. These oligomerization process conditions which favor the production of $C_{10}$-$C_{20}$ and higher aliphatics tend to convert only a small portion of ethene as compared to $C_{3+}$ olefins.

The present invention is based on the observation that methanol, DME or the like may be converted into liquid fuels, particularly distillate, in a multi-stage process with integration between the major process stages providing an alkene-rich recycle stream. The initial stage MTO-type process hydrocarbon effluent stream, after byproduct water separation, can be fed to the MOGD stage for conversion into heavier hydrocarbons. Ethene and $C_5^+$ hydrocarbons may be recovered by interstage separation from the primary effluent or the secondary oligomerization stage gasoline product may be recovered, for recycle to the MTO unit. Advantageously, the recycle is found to be reactive in the presence of ZSM-5 type zeolite-containing catalysts and increase the yield of $C_3$-$C_4$ olefins and, ultimately, the overall distillate yield. This procedure is particularly useful for converting recycled gasoline in a fluidized bed MTO unit, and/or for recycling virtually to extinction the ethene component of the primary stage effluent.

According to the invention, there is provided a process for converting a feedstock comprising methanol, dimethyl ether or mixtures thereof into distillate range liquid hydrocarbons comprising the steps of

(a) contacting the feedstock with a catalyst comprising a crystalline zeolite in a first catalyst stage, at an elevated temperature and a moderate pressure to convert the feedstock into hydrocarbons comprising $C_2$-$C_4$ olefins and $C_5^+$ hydrocarbons;

(b) cooling the effluent from step (a) and separating the cooled effluent to obtain an aqueous liquid stream, a heavy hydrocarbon liquid stream and a light hydrocarbon vapor stream rich in $C_2$-$C_4$ olefins;

(c) compressing the light hydrocarbon vapor stream from step (b) to obtain an olefinic stream rich in $C_3^+$ olefins and a vapor stream rich in ethene;

(d) compressing and contacting the olefinic liquid stream from step (c) in a second catalyst stage with an oligomerization catalyst comprising a medium-pore, shape-selective, acidic zeolite at a substantially increased pressure and moderate temperature to convert at least a portion of the olefins into a heavier liquid hydrocarbon product stream comprising olefinic gasoline and distillate range liquids; and

(e) recycling to step (a) at least a portion of the olefinic gasoline from step (d) and/or at least a portion of the ethene from step (c).

Advantageously, the first and second stage catalyst comprise ZSM-5 type zeolite. In the first stage, conversion is conducted over an HZSM-5 zeolite catalyst to provide a light olefinic hydrocarbon vapor stream comprising a major amount of $C_3$–$C_4$ olefins and a minor amount of ethene. Preferably, when gasoline is recycled to the first stage, it is recycled on an amount of 1 to 30 parts gasoline ($C_5$–$C_9$) per 100 parts by weight of hydrocarbon equivalent in the feedstock. Sililarly, when ethene is recycled to the first stage, it is preferably recycled in an amount of 1 to 20 parts ethene per 100 parts hydrocarbon equivalent in the feedstock. By fractionating the gaseous effluent separated from the first stage effluent, a light recycle gas stream may be recovered containing at least 90% of ethene from the first catalyst stage and an olefinic stream rich in $C_3{}^+$ olefins.

Other objects and features of the invention will be seen in the following description and drawings.

Numerous oxygenated organic compounds may be contained in the feedstock passed to the first catalyst stage. Since methanol and its ether derivative (DME) are industrial commodities readily available from synthesis gas or the like, these materials are preferred starting materials. It is to be understood, however, that MTO-type processes can employ methanol, dimethylether and mixtures thereof, as well as other aliphatic alcohols, ethers, ketones and/or aldehydes. It is known that oxygenates can be partially converted by dehydration, as in the catalytic reaction of methanol over gamma-alumina to produce DME. Typically, an equilibrium mixture of methanol, DME and water is produced by partial dehydration. This reaction takes place in either conversion of methanol to lower olefins (MTO) or methanol to gasoline (MTG).

Catalyst versatility permits the same zeolite to be used in both the first stage (MTO) catalyst and second oligomerization stage (MOGD) catalyst. While different catalytically acitve materials may be used in these two stages, it is advantageous to employ catalysts comprising a standard ZSM-5 having a silica-to-alumina molar ratio of 70:1, for example.

The preferred oligomerization catalysts comprise a crystalline aluminosilicate zeolite having a silica to alumina ratio of at least 12, a constraint index of 1 to 12 and acid cracking activity of 160 to 200. Representative of such ZSM-5 type zeolites are ZSM-5, ZSM-11, ZSM-12, ZSM-22, ZSM-23, ZSM-35 and ZSM-38. ZSM-5 is described in U.S. Patent 3,702,886 and U.S. Patent Re. 29,948; ZSM-11 is described in U.S. Patent 3,709,979; ZSM-12 is described in U.S. Patent 3,832,449; ZSM-23 is described in U.S. Patent 4,076,842; ZSM-35 is described in U.S. Patent 4,016,245; and ZSM-38 is described in U.S. Patent 4,046,839. An especially suitable catalyst for fixed bed operations comprises HZSM-5 zeolite with 35 weight % alumina binder in the form of cyclindrical extrudates of about 1 to 5mm diameter. These medium pore shape selective zeolites are sometimes known as porotectosilicates or "pentasil" materials.

Other catalysts and processes suitable for converting methanol/DME into lower olefins are described in U.S. Patents 4,393,265 and 4,387,263, and European Patent Application 0081683. Another suitable zeolite is ZSM-45. In addition to the preferred aluminosilicates, borosilicate, ferrosilicate and "silicalite" materials may be employed. ZSM-5 type zeolites are particularly advantageous because the same material may be employed for dehydration of methanol into DME, conversion to lower olefins and oligomerization.

Carrying out the process of the invention, various reactor configurations may be used, including fluidized bed catalytic reactors, moving bed and fixed bed reactors.

The process of the invention will now be described in greater detail by way of example only with reference to the accompanying drawings, in which

FIG. 1 is a process flow sheet showing the major unit operations and process streams;

FIG. 2 is a schematic representation of a preferred inter-stage separation system for ethene recovery;

FIG. 3 is a schematic representation of an alternative system;

FIG. 4 is a process flow sheet of an integrated olefins upgrading process; and

FIG. 5 is an alternative process flow sheet depicting optional integration techniques.

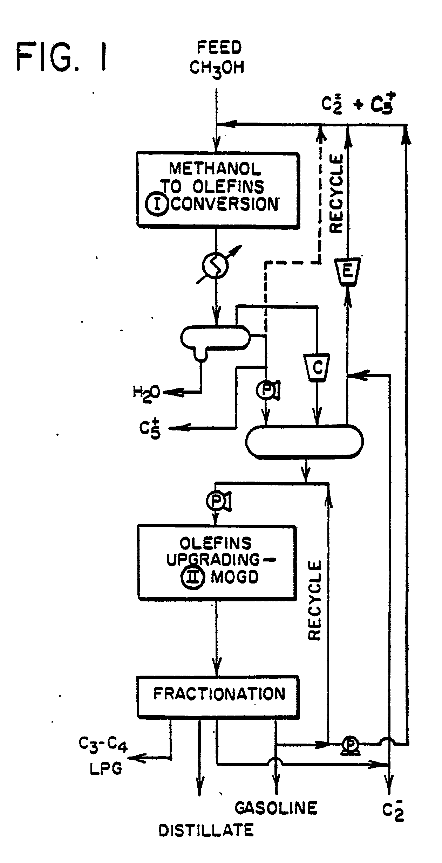

Referring to Fig. 1 of the drawings, the process feedstock (methanol and/or DME, for example) is fed to the first conversion stage I where it is converted into lower olefins and gasoline hydrocarbons plus water by dehydration. Byproduct water is recovered by simple phase separation of the cooled effluent. Liquid hydrocarbons consisting essentially of $C_5{}^+$ gasoline boiling range materials may be recovered or pumped to the higher secondary stage pressure, or optionally recycled to the first stage reactor. At least a portion of the vapor phase effluent from the first stage is compressed and heated along with gasoline diluent or throughput liquids to oligomerization reaction temperature, and the combined olefinic stream (optionally containing recycled olefinic gasoline) is reacted in second stage II at high pressure and elevated temperature over the catalyst. Second stage II effluent is then separated into light gases, $C_5{}^+$ gasoline for recycle in part to the first stage and second stage reactors, and distillate range products. The distillate stream comprises a major fraction of $C_{10}$–$C_{20}$ high boiling aliphatics and may contain a minor amount of aromatics.

In the process for the catalytic conversion of olefins into heavier hydrocarbons by oligomerization using a catalyst comprising an acid crystalline zeolite, such as ZSM-5 type zeolite, process conditions can be varied to favor the formation of either gasoline or distillate boiling range products. At moderate temperature and relatively high pressure, the conversion conditions favor distillate range products having a normal boiling point of at least 165°C. Lower olefinic feedstocks containing $C_2$–$C_6$ alkenes may be converted selctively; however, the distillate mode conditions do not convert a major fraction of ethene. While propene, butene-1 and others may be converted to the extent of 50 to 99% in the distillate mode, only about 10 to 50% of the ethene will be consumed.

Alkylation of ethene with methanol over ZSM-5-containing catalysts has been described by Kaeding et al (J. Catalysis; Jan. 1980, Aug. 1984), and it is known to recycle ethene in the production of aromatic gasoline from methanol over zeolites (U.S. Patent 3 998 899). In a fluidized bed plant for converting methanol into lower olefins and gasoline, recycle of ethene at a rate of 2.5 parts by weight be 100 parts $CH_2$ equivalent in the feedstock methanol provides a product yield that is substantially the same, as shown in Table I. These continuous runs are conducted under the same conditions.

<div align="center">

TABLE I

Hydrocarbon Product Yield, Wt %

</div>

| Component | Without Recycle | With Ethene Recycle |
|---|---|---|
| $C_1$ | 0.8 | 0.8 |
| $C_2$ | 0.3 | 0.3 |
| $C_2=$ | 2.5 | 2.7 |
| $C_3$ | 4.4 | 4.5 |
| $C_3=$ | 4.6 | 4.5 |
| $nC_4$ | 2.1 | 2.1 |
| $iC_4$ | 10.8 | 10.4 |
| $C_4=$ | 5.4 | 5.1 |
| $C_5+$(Gasoline) | 69.1 | 69.6 |
| Total | 100.0 | 100.0 |

$T = 407°C,$ $P = 400KPa,$ $WHSV = 2.65 \ hr^{-1}$ (based on HZSM-5).

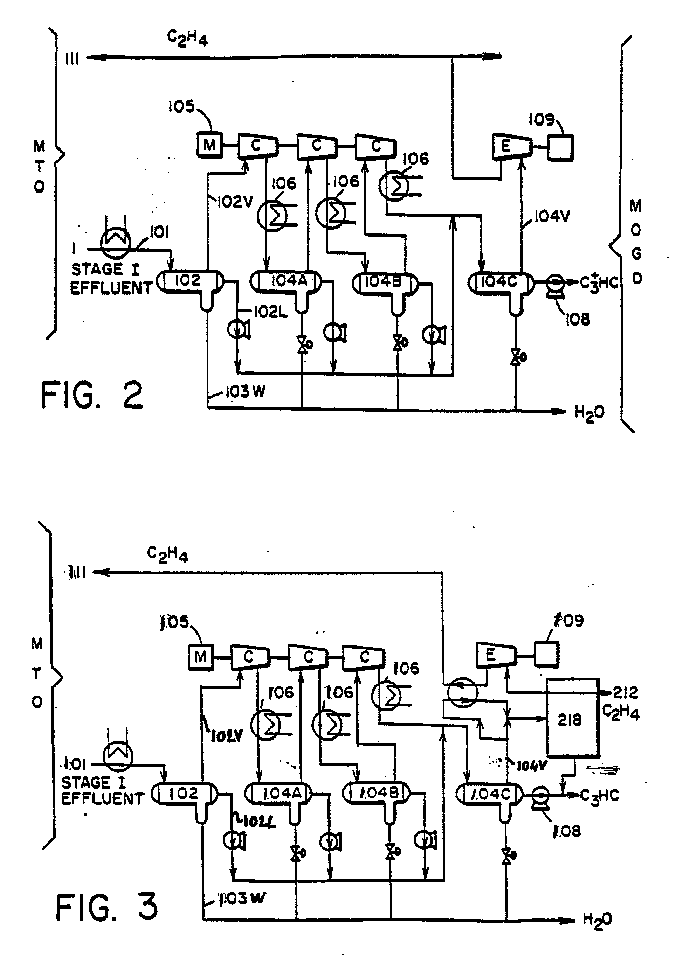

Referring now to Fig. 2, the light hydrocarbon vapor stream separated from the first stage effluent is compressed in a plurality of compression stages to condense liquid olefinic hydrocarbons (HC). The full reaction effluent of the first stage MTO plant is passed via conduit 101 and primary phase separator 102 to provide a first vapor stream 102V rich in $C_4$-hydrocarbons, liquid hydrocarbons stream 102L, and by product water stream 103W. The liquid (eg-$C_5+$) stream 102L is combined with a corresponding liquid HC from succeeding separators and withdrawn. The primary vapor stream 102V is adiabatically compressed by multi-stage motor-compressor set 105 M–C, cooled via exchanger 106 and passed to a succeeding separator 104A, at which point the preceeding phase separation technique is repeated. Similarly, other separators 104B and 104C operate to provide an ethene-rich recycle stream 104V, which is passed to turbo-expander 109E and thus at MTO pressure back vial line 111 to the olefins production in the first stage. Advantageously, the MTO effluent is received at about atmospheric pressure (for example, 100 to 150 kPa) and compressed in plural stages to an intermediate pressure of about 1100 to 3500 kPa and separated in a final vessel 104C at about ambient temperature (20 to 60°C). Olefinic liquids rich in $C_3+$ aliphatic hydrocarbons are recovered from the final compressor stage via pump 108 which pressurizes the liquid HC stream to sufficiently high pressure for it to be employed in the following secondary stage MOGD unit.

A further modification of the interstage ethene separation technique described above is depicted in the flow diagram in Fig. 3, in which corresponding apparatus and process streams are identified by corresponding numbers to those of Fig. 2. In this modification, ethene-rich vapor withdrawn from the separator 104C via line 104V is cooled by heat exchange and further processed to increase ethene purity in ethene unit 218. It will be understood that ethene can be treated in a cryogenic plant cold box, de-ethanizer tower, absorption unit or the like to remove undesirable components prior to recycle 111 and/or recovery 212. A suitable selective sorption unit is described in U.S. Patent 4 471 147. Preferably, compressed light hydrocarbons are fractionated to recover a recycle stream containing at least 90 mole percent ethene. This can be achieved by selectively absorbing $C_3^+$ components in a $C_5^+$ liquid hydrocarbon sorbent stream.

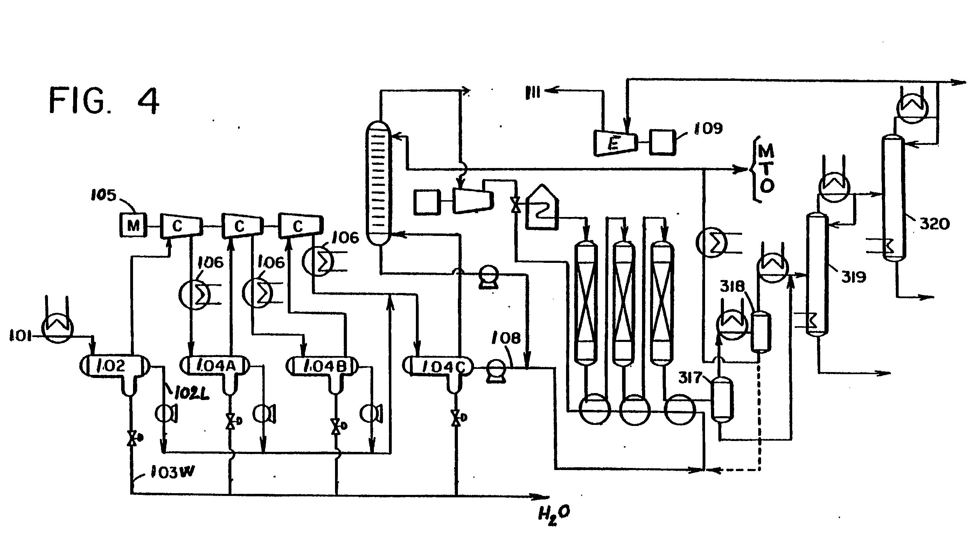

In Fig. 4 a continuous multi-stage catalytic system is depicted for converting oxygenated feedstock into liquid hydrocarbons. The process flow diagram shows an integrated plant. The first stage includes a catalytic reactor containing acidic zeolite catalyst for converting oxygenate to olefinic hydrocarbons rich in $C_2$–$C_4$ alkenes. The interstage section includes separation stages for recovering water and light hydrocarbon vapor from the first stage effluent stream, compressors for pressurizing the first stage hydrocarbon effluent stream to recover an intermediate hydrocarbon liquid stream rich in $C_3^+$ components and an ethene-rich vapor stream. The second stage includes catalytic oligomerization reactor containing medium pore shape selective acidic zeolite oligomerization catalyst for converting the $C_3^+$ olefinic hydrocarbons into heavier liquid hydrocarbons. Franctionation towers 317, 318, 319, 320 enable separation of second stage effluent into a light hydrocarbon stream containing $C_2$–$C_4$ aliphatic hydrocarbons, a $C_5^+$ gasoline stream and distillate range stream. By recovering and recycling at least a portion of the ethene and/or gasoline to the first stage for reconversion, an economic system is achieved. The $C_5$ to $C_9$ gasoline range olefins are coproduced with varying amounts of other hydrocarbons in this boiling range, especially aromatics. While aromatics are not made in large quantity in the MOGD second reactor stage due to relatively low operating temperatures, the first stage MTO reactor can produce significant quantities at further elevated temperatures. By separating the heavier ($C_8^+$) aromatics from the MTO effluent, a valuable high octane liquid fuel may be reoovered and formation of undesirable byproducts avoided.

Gasoline boiling range hydrocarbons from the second stage MOGD effluent typically contain at least 50 weight % to $C_5$ to $C_9$ olefins, with 90% or more mono-alkene being obtainable. These are mostly straight chain or slightly branched mono-olefinic molecules. A large portion of this effluent stream may be recycled to the MOGD reactor system for upgrading to distillate. The process of the invention is able to reconvert a recycled portion of the MOGD gasoline by introducing it to the first stage reactor. At the higher temperature (for example 475°C+), the aliphatic hydrocarbons undergo cracking and interpolymerization to produce a widely-distributed equilibration product range comprising $C_2^=$, a large fraction of $C_3^=$–$C_4^=$, and heavier olefins. Recycled aliphatic hydrocarbon is particularly advantageous in the increasing production of propene and butenes.

In view of the highly reactive nature of methanol/DME and other oxygenate feedstocks, it is preferred to isolate the $C_5^+$ recycle reactant stream by introducing it to the MTO reactor substantially downstream from the oxygenate inlet at a point where the oxygenate has been essentially completely converted into hydrocarbons. This feature of the process prevents alkylation of the recycled $C_{5+}$ components by methanol or the like and optimizes net production of the desirable $C_3$-$C_4$ olefins. To facilitate the olefinic gasoline equilibrating reactions from oxygenates, a fluidized bed catalytic MTO reactor is satisfactory. A suitable reactor and operating teohnique are described in U.S. Patent 4,547,616. In the preferred MTO reactor, a bed of finely divided (less than 150 microns) ZSM-5 catalyst is maintained in a turbulent fluidization regime. Hot feedstock vapor is passed upwardly through the fluidized bed at a superficial velocity of 0.3 to 2 meters per second, maintaining a bed density of about 100 to 300 kg/m². Operating at about 520 ± 20°C and a catalyst activity sufficient to yield a propane: propene ratio of 0.02 to 0.3:1, production of ethene can be controlled at a low level.

In the examples below, the aliphatic gasoline recycle stream contains 94 wt% of $C_5$-$C_9$ olefins, 4% $C_5$-$C_{10}$ paraffins and 2% $C_6$-$C_{11}$ aromatics. This recycle stream is produced by a second stage oligomerization unit operating at about 5500 kPa, 1 WHSV (based on weight of fresh olefin feed to ZSM-5 catalyst) at 260°C, using the MOGD system of Fig. 4 with 2:1 internal MOGD gasoline recycle. The portion of this gasoline recycled to the first stage MTO reactor in Case B is introduced at midpoint stratum in the fluidized bed, corresponding to more than 99.5% methanol conversion. In case C the gasoline recycle is combined with the methanol feedstock and introduced at the bottom reactor inlet. Case A is the control run without recycle. The results of three continuous MTO runs are given in Table II, based on 100 parts by weight of hydrocarbon equivalent in the feedstock methanol.

### Table II

#### Material Balance for Aliphatic Gasoline Recycle

|  | Case A | Case B | Case C |
|---|---|---|---|
| $C_1$ | 2.0 | 2.1 | 2.0 |
| $C_2$ | 0.4 | 0.6 | 0.4 |
| $C_2^=$ | 5.2 | 7.4 | 6.4 |
| $C_3$ | 2.1 | 3.4 | 3.2 |
| $C_3^=$ | 32.9 | 38.2 | 34.0 |
| $n-C_4$ | 0.7 | 1.0 | 1.3 |
| $i-C_4$ | 2.3 | 3.1 | 4.7 |
| $C_4^=$ | 19.1 | 22.6 | 20.4 |
| $C_5^+$ P+N | 9.1 | 15.1 | 20.3 |
| $C_5^+$ O | 19.7 | 21.4 | 20.1 |
| Aromatics | 6.5 | 9.6 | 11.7 |
|  | 100.0 | 124.5 | 124.5 |

The above runs are conducted at 500°C, 180 kPa and about 2.1 WHSV. When operating an integrated MTO/MOGD plant according to each of the three cases, A, B, C, above, the net percentage of distillate product is increased by recycling gasoline to the primary stage, as shown in Table III.

### Table III

#### Integrated MTO/MOGD Product Yields

| Product Yields, Wt. % | Case A | Case B | Case C |
|---|---|---|---|
| Fuel Gas | 2.5 | 2.9 | 2.6 |
| LPG | 4.8 | 6.5 | 7.9 |
| $C_5$-175°C Gasoline | 31.7 | 20.0 | 26.0 |
| 175°C$^+$ Distillate | 61.0 | 70.6 | 63.5 |
|  | 100.0 | 100.0 | 100.0 |
| Total gasoline + distillate (G+D) | 92.7 | 90.6 | 89.5 |
| Gasoline Octane (R+O) | 93.2 | 97.5 | 95.1 |

The optimum case B not only increases the distillate yield, but also provides gasoline product of enhanced octane rating. Both cases B and C provide gasoline recyle to the primary stage at a continuous rate of 24.5 parts by weight per 100 parts of hydrocarbon equivalent in the methanol feed. This recycle rate may be varied from about 1 to 30 parts per 100 HC depending on the combined effects of ethene recycle and MOGD operating mode, for example.

When introducing recycle gasoline to the MTO fluidized bed, the recycle stream should be uniformly distributed by injecting vapor or liquid at least 10% up the bed height, preferably 25 to 50% up the bed. It should be understood that more than 99% of the methanol can be converted in the initial 25% of the space time interval. Under the conditions described, methanol conversion into lower olefins and recycle equilibration are maintained essentially separate, with less than 1%, preferably less than 0.2% of the methanol remaining unconverted at the point or stratum of recycle injection.

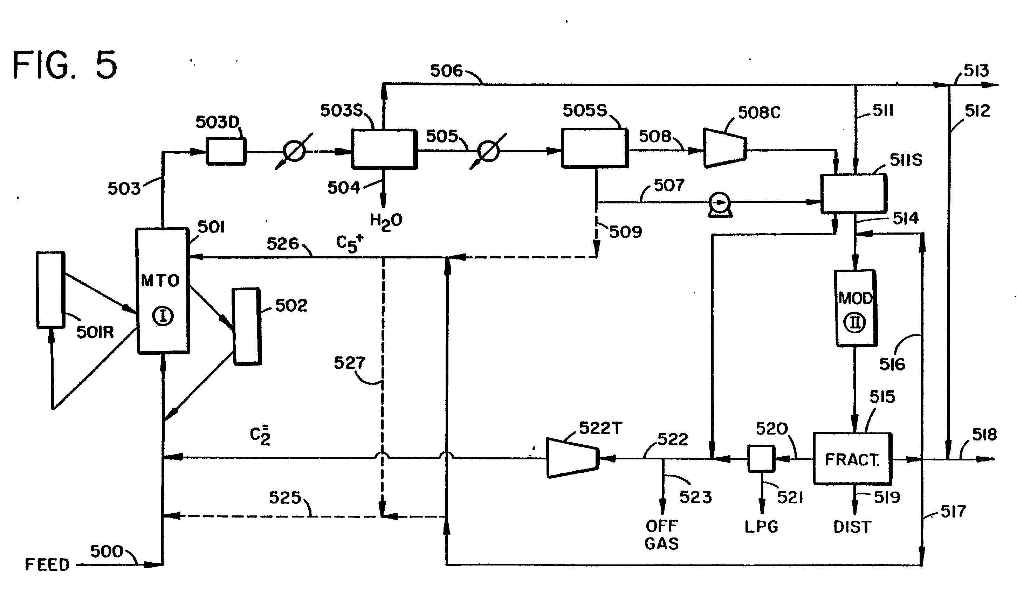

A schematic flow sheet of the system is shown in Figure 5. Vaporized and/or liquid methanol 500 is fed to the fluid bed Stage I MTO reactor 501. The water content of the feed can be 0 to 50 weight %, preferably less than 5 weight %. An ethene rich gas stream 524 can be co-fed with the methanol. The main fluid bed reactor can be operated either in the preferred turbulent dense bed mode or as a dilute phase riser reactor. In this example, exothermic heat of reaction is removed in an external cooling vessel 502 by circulating catalyst through transfer lines. Conversion catalyst is circulated continuously through a regenerator vessel 501R.

If maximum distillate production is desired, an aliphatic gasoline recycle stream 526 is introduced as a vapor and/or liquid directly into the fluid bed reactor 501. Location of the injection point is important. The gasoline recycle should first contact the catalyst in a region where the local concentration of unconverted methanol is low (less than 1 weight %, preferably less than 0.05 weight %) to prevent formation of higher paraffins and aromatics via reaction with methanol.

In the absence of appreciable quantities of methanol, the gasoline recycle stream will equilibrate to a mixture rich in low molecular weight olefins and aromatics. Overall, MTO olefin selectivity will increase with a concommitant increase in stage II distillate production. As an alternative, gasoline recycle streams 509 and 517 may be admixed with fresh methanol feed via conduit 525. The aliphatic/olefinic gasoline so recycled can react with methanol in the MTO reactor 501 to make primarily high octane, aromatic gasoline.

The total MTO product gas stream 503 is cleaned via catalyst separation device 503D, cooled, and separated in vessel 503S into a water stream 504, light hydrocarbon vapor 505 and condensed aromatic gasoline 506. The heavy gasoline 506 can be fed to the Stage II distillate mode MOD reactor via lines 511 and 514, combined with recycle gasoline 516 via line 512, or isolated as a final product 513. The light hydrocarbon vapor stream 505 is cooled further and separated in unit 505S into liquid 507 and light gaseous hydrocarbon stream 508. Liquid hydrocarbons from the low temperature separator 505s are pumped to the Stage II reactor via high pressure separator 511S and/or recycled to the Stage I MTO reactor via line 509. The light hydrocarbons are compressed 508C and sent to the high pressure separator 511S. The pressure and temperature of the separator are chosen so that ethane, ethene, and lighter compounds are in the gaseous phase while the $C_{3+}$ fraction is a liquid. The olefinic liquid hydrocarbons 514 are combined with gasoline recycle stream 516 and pumped to the Stage II fixed bed reactor system. Hydrocarbon gas stream 522, which is predominately ethene, is recycled to the inlet of the fluid bed reactor 501 after energy recovery in a turboexpander 522T. The secondary stage fractionation system 515 is operated in a conventional manner to produce LPG, gasoline range, and diesel range products (streams 521, 518, 519), except that a fraction of the LPG rich, $C_2$-containing off-gas may be recycled to the fluid bed reactor 501 via turboexpander 522T. Purge gas stream 523 is sent to a fuel gas plant to prevent the build-up of paraffins in the recycle loop.

The combined processes are an effective means for converting oxygenated organic compounds, such as methanol, DME, lower aliphatic ketones, aldehydes and esters, into valuable hydrocarbon products. Thermal integration is achieved by employing heat exchangers between various process streams, towers and absorbers.

## Claims

1. A process for converting a feedstock comprising methanol, dimethyl ether or mixtures thereof into distillate range liquid hydrocarbons comprising the steps of

(a) contacting the feedstock with a catalyst comprising a crystalline zeolite in a first catalyst stage, at an elevated temperature and a moderate pressure to convert the feedstock into hydrocarbons comprising $C_2$-$C_4$ olefins and $C_{5+}$ hydrocarbons;

(b) cooling the effluent from step (a) and separating the cooled effluent to obtain an aqueous liquid stream, a heavy hydrocarbon liquid stream and a light hydrocarbon vapor stream rich in $C_2$-$C_4$ olefins;

(c) compressing the light hydrocarbon vapor stream from step (b) to obtain an olefinic stream rich in $C_{3+}$ olefins and a vapor stream rich in ethene;

(d) compressing and contacting the olefinic liquid stream from step (c) in a second catalyst stage with an oligomerization catalyst comprising a medium-pore, shape-selective, acidic zeolite at a substantially increased pressure and moderate temperature to convert at least a portion of the olefins into a heavier liquid hydrocarbon product stream comprising olefinic gasoline and distillate range liquids; and

(e) recycling to step (a) at least a portion of the olefinic gasoline from step (d) and/or at least a portion of the ethene from step (c).

2. A process according to claim 1, wherein the first stage feedstock is converted over a catalyst comprising HZSM-5 zeolite to provide a light olefinic hydrocarbon vapor stream comprising a major amount of $C_3$-$C_4$ olefins and a minor amount of ethene.

3. A process according to claim 1 or claim 2, wherein the ethene rich stream obtained in step (c) contains at least 90% of the ethene from the first catalyst stage.

4. A process according to any one of claims 1 to 3, wherein the first and second stage catalysts comprises ZSM-5 type zeolite and wherein olefinic gasoline is recycled to the first catalyst stage at a rate of 1 to 30 parts gasoline per 100 parts by weight of hydrocarbon equivalent in the feedstock.

5. A process according to any one of claims 1 to 4, wherein in step (c), $C_{3+}$ components are selectively absorbed in a $C_{5+}$ liquid hydrocarbon stream.

6. A process according to claim 5, wherein the $C_{5+}$ liquid hydrocarbon stream comprises olefinic gasoline from step (d).

7. A process according to any one of claims 1 to 6, wherein the recycle stream is injected into the first catalyst stage at a point downstream of the feedstock.

8. A process according to claim 7, wherein the recycle stream is injected downstream of the point at which at least 99% of the feedstock has been converted.

**Patentansprüche**

1. Verfahren zur Umwandlung eines Ausgangsmaterials, das Methanol, Dimethylether oder Mischungen davon umfaßt, in flüssige Kohlenwasserstoffe im Destillatbereich, welches die Schritte umfaßt:

(a) Kontakt des Ausgangsmaterials mit einem Katalysator, der einen kristallinen Zeolith umfaßt, in einer ersten Katalysatorstufe bei erhöhter Temperatur und mäßigem Druck, um das Ausgangsmaterial in Kohlenwasserstoffe umzuwandeln, die $C_2$–$C_4$-Olefine und $C_5^+$-Kohlenwasserstoffe umfassen;

(b) Abkühlen des Abflusses vom Schritt (a) und Abtrennung des abgekühlten Abflusses, um einen wäßrigen flüssigen Strom, einen flüssigen Strom schwerer Kohlenwasserstoffe und einen Dampfstrom leichter Kohlenwasserstoffe, der reich an $C_2$–$C_4$-Olefinen ist, zu erhalten;

(c) Komprimieren des Dampfstromes leichter Kohlenwasserstoffe vom Schritt (b), um einen an $C_3^+$-Olefinen reichen olefinischen Strom und einen Dampfstrom zu erhalten, der reich an Ethen ist;

(d) Komprimieren und Kontakt des olefinischen flüssigen Stroms vom Schritt (c) in einer zweiten Katalysatorstufe mit einem Oligomerisierungskatalysator, der einen sauren formselektiven Zeolith mit mittleren Poren umfaßt, bei wesentlich erhöhtem Druck und mäßiger Temperatur, um mindestens einen Teil der Olefine in einen flüssigen Produktstrom schwerer Kohlenwasserstoffe umzuwandeln, der olefinisches Benzin und Flüssigkeiten im Destillatbereich umfaßt, und

(e) Rückführung zumindest eines Teils des olefinischen Benzins vom Schritt (d) und/oder zumindest eines Teils des Ethens vom Schritt (c) zum Schritt (a).

2. Verfahren nach Anspruch 1, worin das Ausgangsmaterial der ersten Stufe über einem Katalysator umgewandelt wird, der einen Zeolith HZSM-5 umfaßt, um einen Dampfstrom leichter olefinischer Kohlenwasserstoffe zu schaffen, die die Hauptmenge der $C_3$–$C_4$-Olefine und eine geringe Menge des Ethens umfassen.

3. Verfahren nach Anspruch 1 oder 2, worin der im Schritt (c) erhaltene an Ethen reiche Strom mindestens 90% des Ethens aus der ersten Katalysatorstufe enthält.

4. Verfahren nach einem der Ansprüche 1 bis 3, worin die Katalysatoren der ersten und zweiten Stufe einen Zeolith vom Typ ZSM-5 umfassen, und worin das olefinische Benzin in einer Menge von 1 bis 30 Gewichtsteile Benzin pro 100 Gewichtsteile Kohlenwasserstoff-Äquivalent im Ausgangsmaterial zur ersten Katalysatorstufe zurückgeführt wird.

5. Verfahren nach einem der Ansprüche 1 bis 4, worin im Schritt (c) die $C_3^+$-Komponenten selektiv im flüssigen $C_5^+$-Kohenwasserstoffstrom absorbiert werden.

6. Verfahren nach Anspruch 5, worin der flüssige $C_5^+$-Kohlenwasserstoffstrom das olefinische Benzin vom Schritt (d) umfaßt.

7. Verfahren nach einem der Ansprüche 1 bis 6, worin der Kreislaufstrom an einem Punkt stromabwärts zum Ausgangsmaterial in die erste Katalysatorstufe eingespritzt wird.

8. Verfahren nach Anspruch 7, worin der Kreislaufstrom stromabwärts des Punktes eingespritzt wird, an dem mindestens 99% des Ausgangsmaterials umgewandelt sind.

**Revendications**

1. Un procédé pour convertir une charge d'alimentation comprenant du méthanol, de l'éther diméthylique ou leurs mélanges en hydrocarbures liquides de la gamme du distillat, caractérisé en ce qu'il comprend les étapes suivantes:

(a) la mise en contact de la charge d'alimentation avec un catalyseur comprenant une zéolite cristalline dans un premier étage de catalyseur, à une température élevée et à une pression modérée pour convertir la charge d'alimentation en hydrocarbures comprenant des oléfines en $C_{2-4}$ et des hydrocarbures en $C_5^+$;

(b) le refroidissement de l'effluent provenant de l'étape (a) et la séparation de l'effluent refroidi pour obtenir un courant liquide aqueux, un courant liquide d'hydrocarbures lourds et un courant de vapeur d'hydrocarbures légers riche en oléfines en $C_{2-4}$;

(c) la compression du courant de vapeur d'hydrocarbures légers provenant de l'étape (b) pour obtenir un courant oléfinique riche en oléfines en $C_3^+$ et un courant de vapeur riche en éthène;

(d) la compression et la mise en contact du courant liquide oléfinique provenant de l'étape (c) dans un second étage de catalyseur, avec un catalyseur d'oligomérisation comprenant une zéolite acide à pores moyens, sélective quant à la forme, à une pression notablement augmentée et à une température modérée pour convertir au moins une partie des oléfines en un courant de produits hydrocarbonés liquides plus lourds comprenant une essence oléfinique et des liquides de la gamme du distillat, et

(e) le recyclage vers l'étape (a) d'au moins une partie de l'essence oléfinique provenant de l'étape (d) et/ou d'au moins une partie de l'éthène provenant de l'étape (c).

2. Un procédé suivant la revendication 1, caractérisé en ce que la charge d'alimentation du premier étage est convertie sur un catalyseur comprenant une zéolite HZSM-5 pour fournir un courant de vapeur d'hydrocarbures oléfiniques légers comprenant une quantité majeure d'oléfines en $C_{3-4}$ et une quantité mineure d'éthène.

3. Un procédé suivant la revendication 1 ou 2, caractérisé en ce que le courant riche en éthène obtenu dans l'étape (c) contient au moins 90% de l'éthène provenant du premier étage de catalyseur.

4. Un procédé suivant l'une quelconque des revendications 1 à 3, caractérisé en ce que les catalyseurs des premier et second étages comprennent une zéolite de type ZSM–5 et que l'essence oléfinique est recyclée dans le premier étage de catalyseur à raison de 1 à 30 parties d'essence pour 100 parties en poids d'équivalent hydrocarbure dans la charge d'alimentation.

5. Un procédé suivant l'une quelconque des revendications 1 à 4, caractérisé en ce que dans l'étape (c), des composants en $C_3^+$ sont absorbés sélectivement dans un courant d'hydrocarbures liquides en $C_5^+$.

6. Un prodédé suivant la revendication 5, caractérisé en ce que le courant d'hydrocarbures liquides en $C_5^+$ comprend de l'essence oléfinique provenant de l'étape (d).

7. Un procédé suivant l'une quelconque des revendications 1 à 6, caractérisé en ce que le courant de recyclage est injecté dans le premier étage de catalyseur à un point situé en aval de la charge d'alimentation.

8. Un procédé suivant la revendication 7, caractérisé en ce que le courant de recyclage est injecté en aval du point auquel au moins 99% de la charge d'alimentation ont été convertis.

FIG. I

FIG. 2

FIG. 3

FIG. 4

EP 0 190 816 B1

FIG. 5

EP 0 190 816 B1